# EUROPEAN PATENT APPLICATION

(11) **EP 4 343 000 A1**
(43) Date of publication of application: **27.03.2024**
(21) Application number: 22197523.8
(22) Date of filing: 23.09.2022
(51) Int. Cl.: C12Q 1/6844, C12Q 1/70

(54) **LIBIHRD (LIQUID BIOPSY HPV RAPID DETECTION) ASSAY**

(71) Applicant: Universität zu Köln, 50923 Köln (DE)
(72) Inventor: Jain, Rishabh, 53115 Bonn (DE); Hübbers, Christian Ulrich, 50997 Köln (DE); Würdemann, Nora, 50678 Köln (DE); Siefer, Oliver Gerhard, 50354 Hürth Hermülheim (DE); Klußmann, Jens Peter, 50825 Köln (DE); George, Julie, 50825 Köln (DE)
(74) Representative: Simmons & Simmons LLP (Munich)

(57) **Abstract**

The present invention relates to a method for determining the presence of human papilloma virus (HPV) in a body fluid, comprising the steps of:
a) Providing a sample of body fluid,
b) Performing RPA with said sample using an E6 and / or an E7 specific primer,
c) Incubating the product of step b) with a CRISPR/Cas complex comprising an E6 and / or E7 specific guide RNA and a Cas protein, and
d) Determining whether a cleavage of E6 and / or E7 DNA has occurred.

## Description

### BACKGROUND OF THE INVENTION

Human papilloma viruses (HPV) are small double-stranded DNA viruses, whose infections commonly cause skin or mucous membrane growths. Some HPV infections cause lesions in the anogenital tract or the oronasopharynx. Some types of HPV infection can even lead to different severe types of squamous cell carcinoma, for example cancer of the lower part of the uterus that connects to the vagina (cervix), anal cancer, penile cancer, oral cancer, oropharyngeal cancer, and several other types of cancers. Until now, there are more than 200 varieties of human papillomaviruses described. Related to squamous cell carcinoma, the 14 most cancer-causing HPV types include types 16, 18, 31, 33, 35, 39, 45, 51, 52, 56, 58, 59, 66 and 68, referred to as high-risk (HR) HPC types.

HPV-induced oropharyngeal squamous cell carcinoma (OPSCC) remains increasing worldwide. Recent data from the U.S. now even show that the number of newly diagnosed OPSCC patients exceeds that of cervical carcinomas. HPV infections are often transmitted sexually or through other skin-to-skin contact.

Known risk facts of most common HPV infections including HR HPV-variants include for example personal contact or damaged skin parts, in particular for skin-to-skin infections, weakened immune system, Cannabis consumption, smoking. alcohol consumption or number of sexual partners, in particular for sexually transmitted virus infections. Nevertheless, other risk factors remain to be explored. Of note, HR HPV variants are of particular clinical relevance, with early prognosis and monitoring of progression and malignant transformation of the disease becoming particularly highly relevant for patients.

In the past decades, vaccines have been proposed as a promising tool for protecting against the strains of HPV most likely to cause cervical and oropharyngeal cancer. Nevertheless, vaccination against the most oncogenic HPV types is unlikely to be as successful as hoped, at least in the near future, so HPV-induced tumors are likely to remain of major medical importance for many years to come. The reasons for this development can be found, for instance, in low vaccination rates worldwide, as well as the population dynamics with an increasing number of elderly people and the merely prophylactic effect of the vaccine, which cannot prevent the progression of temporarily infected tissue to a tumor, which can last for decades. Even though success has been made in detection of HPV infections, existing detection methods are insufficient to meet the clinical requirements, especially in the oropharynx, where precursor lesions are absent. Until today, detection and prognosis as well as surveillance of progression and remission of the disease is quite limited today. Of note, existing detection methods are in particular insufficient as far as diagnosis of HPV-related carcinoma and recurrent disease during follow-up is concerned. Known clinical screening and imaging methods are additionally insufficient because of their detection limits. The recommended gold standard applying p16^{INK4a} immunohistochemistry as a single marker appears insufficient to indicate HPV-driven OPSCC patients with the option to further select those patients suitable for treatment de-escalation since it implies, for example the risk of including such patients with a p16^{INK4a}-positive but HPV DNA-negative tumor. Furthermore, this biopsy-dependent method is not suitable for close follow-up screening.

Thus, fast, reliable, cost-effective, and easy-to-use tests for valid detection of HPV circulating nucleic acids are urgently needed in the detection of cancer to allow for point-of-care (POC) testing.

### SUMMARY OF THE INVENTION

Here, an easy, fast and reliable alternative detection method to overcome the above mentioned limitations regarding efficient detection of clinically relevant HPV infections and HPV related carcinomas is provided. The method relies on the combination of different methods, which are partially known. In detail, provided herein are methods for fast, reliable, cost-effective and easy-to-use tests for valid detection of HPV circulating nucleic acids as well as kits and their use for diagnosis and/or monitoring of HPV infections and/or cancer.

In particular provided herein are methods for determining the presence of human papilloma virus (HPV) in a body fluid, comprising the steps of:
a) Providing a sample of body fluid,
b) Performing RPA with said sample using an E6 and / or an E7 specific primer,
c) Incubating the product of step b) with a CRISPR/Cas complex comprising an E6 and / or E7 specific guide RNA and a Cas protein, and
d) Determining whether a cleavage of E6 DNA and / or E7 DNA has occurred.

Moreover, provided herein is a kit, comprising means for detection cleavage of E6 DNA and / or E7 DNA in a body bluid as stated above and use of the kit for the diagnosing and/or monitoring of cancer.

### DETAILED DESCRIPTION OF THE INVENTION

In one aspect, provided herein is a method for determining the presence of human papilloma virus (HPV) in a body fluid, comprising the steps of:
a) Providing a sample of body fluid,
b) Performing RPA with said sample using an E6 and / or an E7 specific primer,
c) Incubating the product of step b) with a CRISPR/Cas complex comprising an E6 and / or E7 specific guide RNA and a Cas protein, and
d) Determining whether a cleavage of E6 and / or E7 DNA has occurred.

More than 200 human papilloma viruses are known until today. Being the most clinically relevant viruses, HPV16 and HPV18 are known to significantly increase the risk of a number of carcinomas. Thus, in a preferred embodiment, the human papilloma virus (HPV) is HPV16 or HPV18. As stated above, there are other HPV variants, which are capable of inducing several types of cancer. Thus, in another embodiment, the human papilloma virus (HPV) is a righ-risk HPV strain. In another embodiment, the human papilloma virus (HPV) is a low-risk HPV strain. In one preferred embodiment, the human papilloma virus (HPV) is HPV33, HPV35, HPV6 or HPV11.

Determining HPV infections is theoretically possible in every body fluid. Thus, in one embodiment of the present invention, the body fluid is a nasopharyngeal swab. In another embodiment, the body fluid is an oral swab. In another embodiment, the body fluid is saliva. In another embodiment, the body fluid is urine. In a further embodiment, the body fluid can be mucus. In a preferred embodiment, the body fluid is blood.

The body fluid may, in principle, be derived from any animal, because the method disclosed herein is universally applicable. In a preferred embodiment, the body fluid is derived from a human subject.

"Amplification", as it is used commonly in the art means the production of multiple copies of a sequence of DNA. Amplification, as defined above, can be performed by any method in the art. Thus, as an alternative to RPA as disclosed herein, the amplification of DNA can be performed by using polymerase chain reaction (PCR), as it is commonly known in the art for decades.

As claimed herein, the DNA amplification is performed by using Recombinase Polymerase Amplification (RPA). RPA is an isothermal nucleic acid amplification technology, relying on the principle of isothermal enzymes. The RPA reaction exploits enzymes known as recombinases, which form complexes with oligonucleotide primers and pair the primers with their homologous sequences in duplex DNA. A single-stranded DNA binding (SSB) protein binds to the displaced DNA strand and stabilizes the resulting D loop. DNA amplification by polymerase is then initiated from the primer, but only if the target sequence is present. Once initiated, the amplification reaction progresses rapidly, so that starting with just a few target copies of DNA, the highly specific DNA amplification reaches detectable levels within minutes.

Using RPA provides several advantages: First, RPA provides a very fast way of amplification as there is no need for modifying the temperature of the sample in order to produce amplification products. RPA works at optimum reaction temperatures at a broad spectrum of about 37-42°C. Moreover, RPA is highly sensitive and provides high specificity rates. RPA can detect single copies of DNA and tens of copies or fewer of RNA in complex samples, without the need for prior nucleic acid purification, providing additionally time saving. As RPA is performed isothermal, no need for melting of the DNA is needed, as it is commonly done within a PCR reaction. Thus, the reaction can be performed at room temperate, without any further complex machineries. Giving these advantages, RPA provides the first step of a fast, versatile and cheap determination method of the present invention. Of note, all RPA kits that are commercially available can be used in the context of the present invention.

HPV-E6 and -E7 of HPVs at risk for developing carcinoma are both oncogenes, playing a major role in the establishment and the development of human HPV infections (Scheffner et al., Cell, 1990). In particular, initial establishment and subsequent progression of OPSCC or cervix cancer are completely dependent on these two major oncogenes E6 and E7, which are expressed constitutively leading to tumorigenesis. Both proteins are encoded by oncogenic human papillomavirus types such as 16 and 18 and are mandatorily expressed in HPV-associated cancers. These oncoproteins have pleiotropic functions, such as regulation of the cell cycle towards increased proliferation, inhibition of apoptosis, metabolic reprogramming towards anabolic reactions, upregulated telomerases and deregulated chromosomal stability. These functions are well established and can be used to transform established cell lines due to immortalization of primary cell lines. Thus, the viral E6 and E7 oncoproteins are necessary for malignant conversion.

Previous detection methods relied, for example, on the detection of the HPV-L1 gene. HPV-L1 (papillomavirus major capsid protein) is a viral protein with the ability to spontaneously self-assemble into virus-like particles (VLPs). Of note, it has been shown, that viral integration can lead to a loss of the L1 gene (Wagatsuma et al.,1990,J. Virol; Zhao, J-W et al., 2016, BMC), suggesting that using E6 and E7 gene detection is beneficial. Accordingly, amplifying E6 and E7 DNA or DNA fragments especially by RPA represent a further advantageous feature of the present invention.

According to the present invention, primers are used to amplify specifically E6 and / or E7 DNA. The sequence of E6 is shown in SEQ ID NO:1, the sequence of E7 in SEQ ID NO:2. In one embodiment, the specific forward primer for amplifying E6 comprises or consists of the sequence of SEQ ID NO: 3. In one embodiment, the specific reverse primer for amplifying E6 comprises or consists of the sequence of SEQ ID NO: 4. In another embodiment, the specific forward primer for amplifying E7 comprises or consists of the sequence of SEQ ID NO: 5. In another embodiment, the specific reverse primer for amplifying E7 comprises or consists of the sequence of SEQ ID NO: 6.

In one embodiment, the E6 specific primer has the following sequence(s): E6: HPV16-E6 Forward GCACCAAAAGAGAACTGCAATG; HPV16- E6 Reverse GTTTGCAGCTCTGTGCATAACTG. In one embodiment, the E7 specific primer has the following sequence(s): HPV16- E7 Forward CAGCTCAGAGGAGGAGGATG HPV16- E7 Reverse GTAATGGGCTCTGTCCGGT. It is understood that the sequence and composition of amplification primers are simply aligned to the sequence of interest. This is one of the methods that the person skilled in the art can easily adapt based on his expertise.

Clustered regularly interspaced short palindromic repeats (CRISPR)-Cas (CRISPR-associated proteins) are part of a prokaryotic adaptive immune system that is represented in most archaea and many bacteria. Briefly said, this system is based on the nuclease activity of so-called (CRISPR-associated) Cas-proteins, providing efficient possibility of cutting foreign DNA or DNA fragments. Moreover, CRISPR enables incorporation of foreign fragments into CRISPR cassettes in the bacterial genome, providing recognition abilities for further infections and establishing a rudimental way of immune system. The CRISPR/Cas system is comprised of a small single stranded nucleotide sequence (also known as guide RNA or gRNA) and so-called Cas proteins with nuclease activity. These Cas proteins are guided by sequence complementary of the guide RNA and the target sequence of DNA to be cut. Numerous diverse Cas proteins are involved in different steps of the processing of CRISPR loci transcripts, cleavage of the target DNA or RNA, and new spacer integration. Cas proteins and their nuclease activity are used in the context of the present invention for cutting amplified DNA or DNA fragments.

In one embodiment of the present invention, the Cas protein is a type II CRISPR/Cas protein. CRISPR/Cas type II proteins comprise any subtype such as II-A, II-B, and II-C. In one specific embodiment, the Cas protein is a Cas9 protein. In another embodiment, the Cas protein is a type V CRISPR/Cas protein. CRISPR/Cas type V proteins comprise any subtype such as V-A and V-B. In a preferred embodiment, the Cas protein is Cas12. In another embodiment, the Cas protein is a type VI CRISPR/Cas protein. CRISPR/Cas type VI proteins comprise any subtype such as VIA, VI-B, VI-C, and VI-D. In one specific embodiment, the Cas protein is a Cas13 protein. In one embodiment, Cas proteins include all Cas proteins that are capable of cutting nucleic acids, single or double-stranded, or that are having (at least partially) nuclease activity. Thus, in one embodiment, the Cas protein cleaves amplified E6 DNA. In another embodiment, the Cas protein cleaves amplified E7 DNA.

In one embodiment of the present invention, the presence of a cleavage product of E6 and / or E7 DNA is detected. By "determination" or "determining", the action or process of identifying the presence of cut DNA is meant. Theoretically, any method can be used for the determination of cut DNA. Determination methods are widely used in the art so far. Non-limiting examples are gel electrophoresis, bioluminescent assays, or fluorescent assays. In particular, when providing analysis of more than one HPV strain, multiplexed assays become relevant. Thus, in one embodiment, multiplexed assays can be used. In one embodiment, fluorescent multiplex assays can be used. In one particular embodiment, the cut DNA is detected by dipstick assays. A dipstick is one of several measurement devices, allowing the determination of present nucleotide acid fragments in a sample. In one preferred embodiment, the cleavage is determined by chromatographic separation via a lateral flow strip.

In another preferred embodiment, the cleavage is determined by target-activated, non-specific single-stranded deoxyribonuclease cleavage utilizing the trans-cleavage site of Cas12 to cleave an artificial, non-target specific ssDNA, respectively labelled 5' and 3' with two different dyes to distinguish cut from uncut product.

A "kit" as it is used herein is to be understood as a set of articles or equipment needed for a specific purpose. Thus, a kit allows a person to easily follow the method of the invention. In one embodiment, the target-activated, non-specific single-stranded deoxyribonuclease cleavage by Cas12 is detected using a kit.

Kits, as they are used in the context of the present invention, can thus comprise Cas proteins, specific guide RNA(s), nucleic acid/ssDNA, standard buffers and reagents for the detection. Reagents for the detection can also be available with an own buffer.

In one embodiment, the labels for the detection can, e.g., be biotin or FITC. In one particular embodiment of the invention, the binding-partners (antibody/ligand) is invariably attached to a chromatography paper. Thus, when the product to be detected is uncut, it's biotin label binds to the first streptavidine band and will be immobilized there. FITC labelling of the opposite end of the product can then be detected using goat anti-FITC antibodies coupled with gold nanoparticles. When the nucleic acid is cut, the piece labelled with FITCs can move to the second binding site independent from biotin/streptavidine binding. A secondary anti-goat antibody is invariably present on the test band site. This secondary antibody in turn binds the goat anti-FITC antibody labeled with gold nanoparticles, which furthermore is bound to the FITC-coupled nucleic acid fragment. Thus, the cut fragment can be detected as a distinct band.

In case that Cas12 is used within the kit, the kit comprises Cas12 proteins, specific guide RNA(s), nucleic acid/ssDNA, standard buffers and reagents for the detection (namely the detection labels biotin and FITC). Accordingly, Cas12 recognizes a PAM (protospacer adjacent motif) and specifically cuts the targeted nucleic acid of interest, which has been amplified by using RPA. As nucleic acid markers, biotin and FITC can be used. The corresponding binding-partners are invariably attached to the chromatography paper. The uncut product is then completely found to the first biotin band. When the nucleic acid is cut, the nucleic acid piece that is labelled with FITC moves to a second binding site, building a second band. Thus, cleaved product is not detected on a first band but on a second. In another embodiment of the present invention, biotin / FITC labels can be exchanged by fluorophore / quencher. A "quencher" as it is used herein is to be understood as a substance, which absorbs excitation energy from a fluorophore.

In one particular embodiment of the present invention, the detection for cut nucleic acid takes place by Anti-FITC labelled gold-nanoparticle visualization.

"Detection means" as used in the context of the present invention refers to a mean that is capable of detection cut nucleic acids. Thus, in the context of the present invention, the detection means can e.g. be a chromatography paper witch invariably attached biotin or FITC. The detection means thus ensure that cut and uncut nucleic acids can be distinguished.

As set out above, Cas proteins are guided by specific single stranded nucleotide sequences, also known as "guide RNAs" or "gRNAs". In one particular embodiment of the invention, the guide RNA for cutting E6 DNA comprises or consists of the sequence UAAUUUCUACUAAGUGUAGAUUGGGUCGCUCCUGUGGGUCC (SEQ ID NO: 7). In one particular embodiment, the guide RNA for cutting E7 DNA comprises or consists of the sequence UAAUUUCUACUAAGUGUAGAUAUCCUCCUCCUCUGAGCUGU (SEQ ID NO: 8). In any case, it is understood that the sequence and composition of the guideRNA are simply aligned to the target sequence. This is one of the methods that the person skilled in the art can easily adapt to a target sequence, based on his expertise.

In a second aspect of the invention a kit is provided herein, comprising means for detecting the cleavage of E6 and/or E7 DNA in a body fluid. Thus, in one embodiment, the kit comprises means for detecting the cleavage of E6 DNA in a body fluid. In another embodiment, the kit comprises means for detecting the cleavage of E7 DNA in a body fluid. In one embodiment, the cleavage is determined using the method of the first aspect of the invention, as defined above.

In a third aspect, the kit above can be used for the diagnosis and/or monitoring of HPV infections in a subject. The subject may be any animal, but is preferably a human subject. The third aspect also includes that the kit above can be used for the diagnosis and/or monitoring of cancer in a subject, preferably in a HPV-related cancer. Thus, in one embodiment of the invention, the cancer can be selected from a non-limiting list consisting of: vulva cancer, vagina cancer, penis cancer, anus cancer, and oropharynx cancer. In a preferred embodiment, the cancer is cervix cancer. In another preferred embodiment, the cancer is oropharyngeal squamous cell carcinoma (OPSCC).

The invention is further described with the help of the following example and figures, which are intended to illustrate, but not to limit the present invention.

### FIGURE LEGENDS

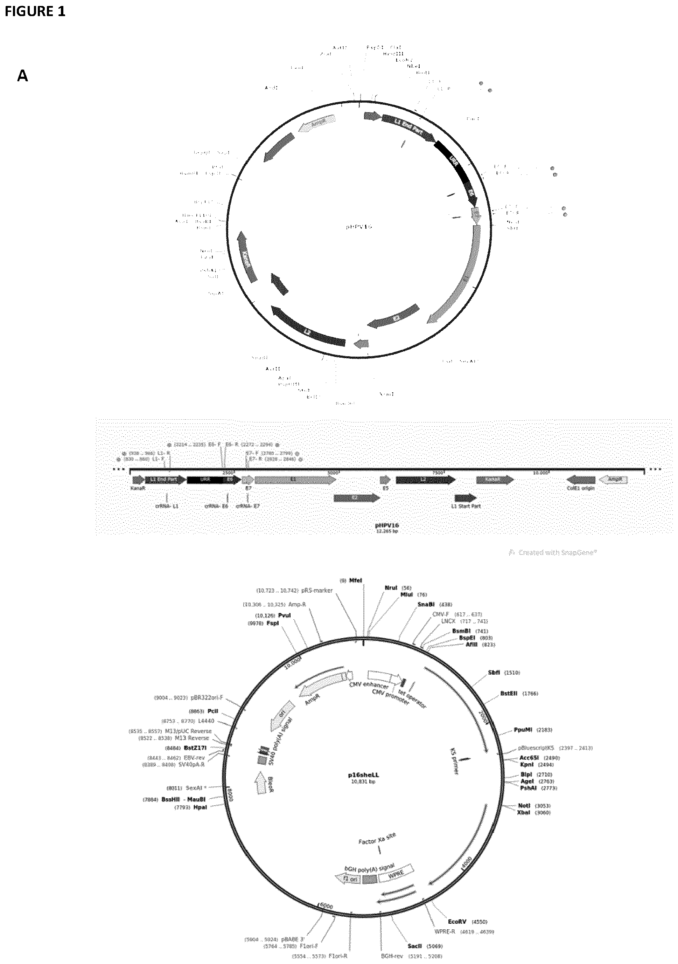

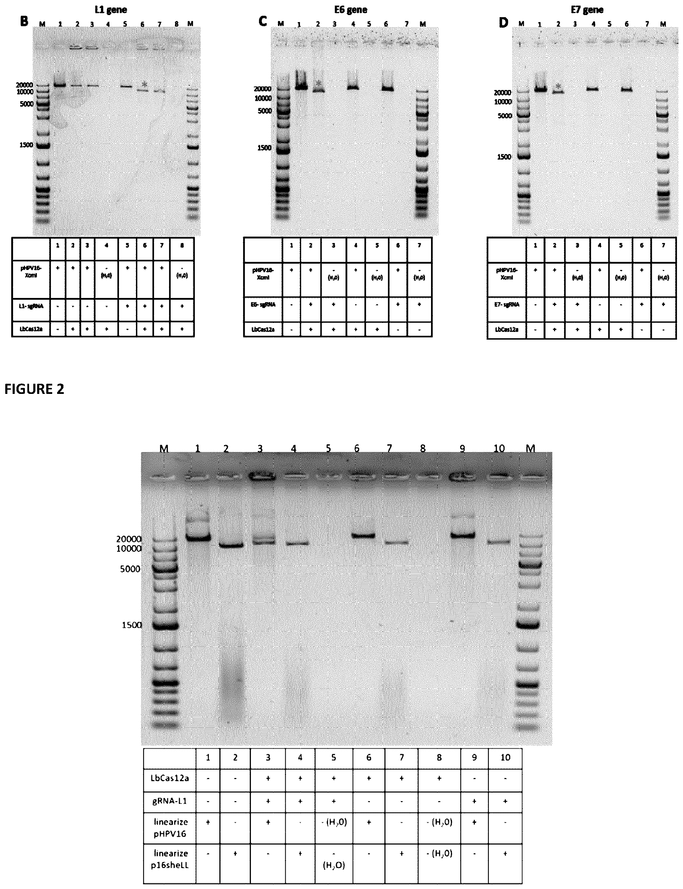

FIGURE 1: LbCas12a-sgRNA cleavage of HPV16 L1/ E6/ E7 genes. (A) HPV16 plasmids and locations of sgRNA targets and universal PCR primers for L1, E6, and E7 genes of HPV16. The LbCas12a protein was in complex with the sgRNAs specific to HPV16-L1/ E6/ E7 genes and was used to cut the linearized HPV16 plasmid. (B) LbCas12asgRNA cleavage of HPV16-L1 gene using L1-sgRNA target at nt 895-914. Lane 6&7 in duplicate. (C) LbCas12asgRNA cleavage of HPV16-E6 gene using E6-sgRNA target at nt 2241-2260. (D) LbCas12a-sgRNA cleavage of HPV16-E7 gene using E7-sgRNA target at nt 2779-2798. The digested sample was loaded onto 1% agarose gel containing 0.5 mg/ ml ethidium bromide concentration covered with 1x TAE buffer. Each lane was loaded with 10 µl of the digested product. The panel (M) indicates the Marker. The expected cleavage products are marked by an asterisk and these bands can be shift lowered compared with the intact linearized DNA.
FIGURE 2: LbCas12a-sgRNA cleavage of L1 gene in HPV16 plasmid and intact linearized product in p16sheLL plasmid taken as a control. The LbCas12a protein was in complex with the sgRNAs specific to the HPV16-L1 gene used to cut the linearized HPV16 plasmid and not with the p16sheLL plasmid. The digested sample was loaded onto 1% agarose gel containing 0.5 mg/ ml ethidium bromide concentration covered with 1xTAE buffer. Each lane was loaded with 10 µl of the digested product. The panel (M) indicates the Marker.

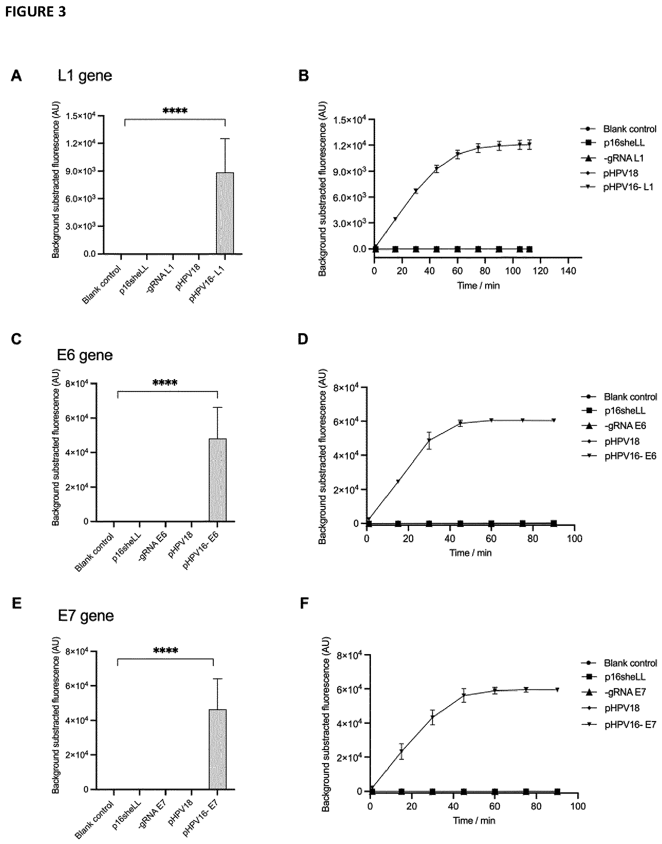

FIGURE 3: Validation of the Cas12a-based HPV16 nucleic acid fluorescence reporting system. The concentrations of the negative control (i.e., non-target dsDNA) and circular HPV16 plasmid were both 1×10⁻⁸ M. Rate of fluorescence recovery and change in fluorescence at the end of the 2 h kinetic study with blank control, nontarget plasmid (p16sheLL), in absence of sgRNA and with circular HPV16 plasmid and change in fluorescence at the end of the 2 h kinetic study for L1 (A, B), E6 (C, D), and E7 (E, F) genes are shown respectively. Background subtracted fluorescence was the fluorescence intensity of the experimental group against the blank control (Targeted nucleic acid was substituted by DNase-free water). Experiments were performed in triplicate (mean ± standard deviation). *, P < 0.05; **, P < 0.01; ***, P < 0.001; ****, P < 0.0001; ns, no significant difference. (A, B) The L1 gRNA was taken as a control and was published in Chen et al., Biotechnology 2018 and Tsou et al., Transl Oncol 2019.

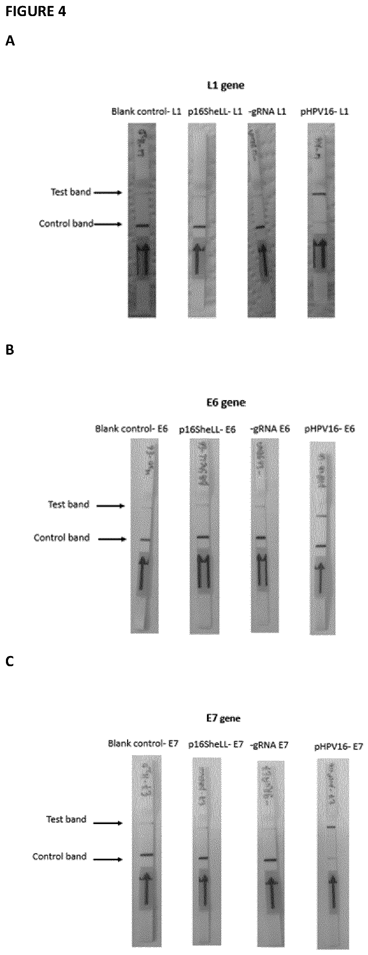

FIGURE 4: Lateral flow detection of HPV16 plasmid along with the negative control, non-target plasmid and in absence of sgRNA for L1 (A), E6 (B) and E7 (C). Blank control, nontarget plasmid (p16sheLL), in absence of sgRNA and with circular HPV16 plasmid are shown for the respective target sequences.

### EXAMPLES

### EXAMPLE 1 - In vitro cutting assay (L1, E6 and E7)

Cas12a cleavage activity guided by L1/ E6/ E7 sgRNA: To validate the specific DNA cutting by the sgRNA-guided LbCas12a, an in-vitro cutting assay was performed. Circular maps of HPV16 and p16sheLL plasmids used for this assay are shown in Figure 1A, respectively. Firstly, the HPV16 plasmid was linearized that contained L1/ E6/ E7 genes with a restriction endonuclease, Xcml, and non-target p16SheLL plasmid with EcoRV to produce linear DNA fragments. Then, the linearized HPV16- L1/ E6/ E7 and p16sheLL plasmid DNAs were cut with LbCas12a nuclease in complex with the sgRNAs specific to HPV16- L1/ E6/ E7 genes. The results confirmed that the HPV16-L1/ E6/ E7 genes could be specifically targeted by their corresponding sgRNAs and cut by the guided LbCas12a nuclease for L1 (Figure 1B), E6 (Figure 1C), and E7 (Figure 1D) genes. Intact linearized product was observed for p16sheLL in lane 4 after cleavage reaction with L1 sgRNA (Figure 2). This means that the in-vitro specific DNA cutting by LbCas12-sgRNA could be used to detect and type DNA.

### EXAMPLE 2- LiBiHRD (Liquid Biopsy HPV Rapid Detection) Assay

LiBiHRD detection protocol works in four steps and can be completed in 1 hour, starting from plasma preparation. Step 1. 5 min incubation: treatment of plasma; Step 2. 20 min incubation: isothermal amplification of the lysis plasma sample using a commercially available recombinase polymerase amplification (RPA) kit; Step 3. 30 min incubation: detection of pre-amplified viral DNA sequence using Cas12; Step 4. 2 min incubation: visual read out of the detection result by eye using a commercially-available paper dipstick.

To check for the presence of HPV-16 in nucleic acid extractions of specimens, two targets were chosen in the HPV-16 genome from the E6 gene and E7 gene. RPA amplification primers and LbCas12a CRISPR guide RNAs were designed for specific detection. In order to maximize the specificity of the assay, guide sequences were selected that minimized off-targets to other HPV genomes. Using serial dilutions of HPV-16 plasmid containing E6/ E7 gene, the presence of HPV-16 DNA sequence in a range between 10-1000 copies per reaction was detectable.

Clinical specimen and plasma preparation: Patient samples should be collected according to the appropriate biosafety procedures. Whole blood from patients was collected before primary treatment. Blood was drawn in commercially available Cell-Free DNA BCT collection tubes (Streck, La Vista, Nebraska, USA) and centrifuged at 515 x g for 10 min at room temperature. Plasma aliquots were stored at -80°C until further analysis. The input for this protocol begins with the lysis of plasma. Collection of plasma samples with routine collection tubes is still matter of optimization.

Reagents: For Step (1) treatment of plasma: Triton X-100 (3051.3) (Carl Roth); PBS (Dulbecco's C-40230) (PromoCell). For Step (2) isothermal amplification: Comercially available TwistAmp^{®} Basic Kit (TABAS03KIT), TwistDx Limited; Specifically designed RPA Amplification primer pairs targeting E6 and E7 gene (synthesized by Metabion). Sequences are additionally shown below.

Target gene amplification RPA primers (5'-->3') HPV16- E6- F GCACCAAAAGAGAACTGCAATG HPV16- E6- R GTTTGCAGCTCTGTGCATAACTG HPV16- E7- F CAGCTCAGAGGAGGAGGATG HPV16- E7- R GTAATGGGCTCTGTCCGGT. For Step (3) detection of HPV16 DNA using Cas12: 10x NEBuffer 2.1 and LbaCas12a (Cpf1), NEB M0653S (New England Biolabs); LbaCas12a crRNA for detecting E6 and E7 gene (synthesized by Metabion). Sequences are additionally shown below.

Target- HPV16 LbCas12a crRNA (5'-->3') E6 gene UAAUUUCUACUAAGUGUAGAUUGGGUCGCUCCUGUGGGUCC E7 gene UAAUUUCUACUAAGUGUAGAUAUCCUCCUCCUCUGAGCUGU; Reporter DNA for lateral flow readout (Lateral-flow reporter: 5'- 6-FAM-TTATT-Bio-3', synthesized by Metabion). For Step (4) reading out using lateral flow dipstick: Commercially available HybriDetect Dipstick (MGHD 1), Milenia Biotec GmbH.

LiBiHRD Protocol Steps:
Step (1): Treatment of plasma: Plasma will be first diluted 1:3 in PBS to avoid solidification. 25 µl of plasma sample will be mixed with 0.53% Triton- X 100 followed by boiling the mixture at 95°C for 5 min.
Step (2): Isothermal Amplification, to be performed in the pre-amplification work area: For testing each sample, set up two RPA reactions, for detection of the E6 and E7 target gene, respectively. In addition, positive controls for E6 and E7 gene can be set up using an HPV16 plasmid. A negative control without test samples should also be included in the setup. For the E6 and E7 gene target (differ by their primers), each reaction can be set up as follows (modified from TwistAmp protocol). Prepare the reaction mix in 1.5 ml tube. Nuclease-free water (ddH₂0) 10.2 µl Primerfree rehydration buffer 29.5 µl E6/ E7- Forward primer (10µM) 2.4 µl E6/ E7- Reverse primer (10µM) 2.4 µl; Add reaction mix to TwistAmp Basic reaction. Pipette to mix and add 3µl of template from step 1. Vortex and spin it briefly; Add 2.5µl of 280mM Magnesium Acetate (MgOAc) and mix well to start the reaction (RPA reaction start as soon as MgOAc is added); Incubate for 4 min at 39°C, then remove the strip from thermomixer, vortex and spin briefly and incubate it for other 16 min at 39°C. Store at 4°C (if tubes are opened after amplification there is a great risk of contamination of work surfaces with amplicon. Ensure that appropriate avoidance measures are taken).
Step (3): Detection of viral DNA sequences using Cas12a. For each E6 and E7 RPA reaction, set up a Cas12a detection reaction as follow: Nuclease-free water 17µl, NEBuffer 2.1 Reaction Buffer (10X) 3µl, 300 nM gRNA 3µl (30 nM final), Lateral flow reporter 10µM 1µl, 1µM EnGen Lba Cas12a (Cpf1) 1µl (30 nM final), Reaction Volume 25µl; Pre-incubate for 10 min at 25°C in thermomixer and add 5µl of RPA template from step 2. After all reactions are set up, vortex to mix thoroughly, spin down in a centrifuge, and incubate at 37°C for 30 min in a thermomixer. After incubation, proceed to Step (4) for lateral flow read out. Step (4): Visual readout of detection result via lateral flow strip. After Step (3), add 80 µl of HybriDetect Assay Buffer to each 20µl reaction and mix thoroughly. Place the diluted reaction in a tube rack at room temperature. Place a HybriDetect Dipstick into each reaction tube and wait for the reaction to flow through the dipstick. Positive control samples should show two lines and negative control samples should only show the bottom line. For each test sample, check to see whether two lines appear for both E6 and E7 genes, indicating a positive HPV16 result.

Optimization: The current protocol is based on commercially available kits giving large experimental flexibility in setting up individual experiments.

### REFERENCES

Scheffner, M. (1990). The E6 oncoprotein encoded by human papillomavirus types 16 and 18 promotes the degradation of p53, Cell, VOLUME 63, ISSUE 6, P1129-1136; https://doi.org)/10.1016/0092-8674(90)90409-8.
Wagatsuma M. (1990). Analysis of integrated human papillomavirus type 16 DNA in cervical cancers: amplification of viral sequences together with cellular flanking sequences, J Virol, Vol. 64, No. 2; doi: 10.1128/jvi.64.2.813-821.1990
Zhao J-W (2016). HPV16 integration probably contributes to cervical oncogenesis through interrupting tumor suppressor genes and inducing chromosome instability; Journal of Experimental & Clinical Cancer Research volume 35, Article number: 180; https://doi.org/10.1186/s13046-016-0454-4.
Chen, J. S., Ma, E., Harrington, L. B., Da Costa, M., Tian, X., Palefsky, J. M., & Doudna, J. A. (2018). CRISPR-Cas12a target binding unleashes indiscriminate single-stranded DNase activity. Science, 360(6387), 436-439.
Tsou, J. H., Leng, Q., & Jiang, F. (2019). A CRISPR test for detection of circulating nuclei acids. Translational oncology, 12(12), 1566-1573.
Piepenburg, O., Williams, C. H., Stemple, D. L., & Armes, N. A. (2006). DNA detection using recombination proteins. PLoS biology, 4(7), e204.

## Claims

1. A method for determining the presence of human papilloma virus (HPV) in a body fluid, comprising the steps of:
a) Providing a sample of body fluid,
b) Performing RPA with said sample using an E6 and / or an E7 specific primer,
c) Incubating the product of step b) with a CRISPR/Cas complex comprising an E6 and / or E7 specific guide RNA and a Cas protein, and
d) Determining whether a cleavage of E6 and / or E7 DNA has occurred.

2. The method of claim 1, wherein the body fluid is selected from the group consisting of blood, nasopharyngeal or oral swabs, saliva, and urine, preferably wherein the body fluid is blood.

3. The method of any of claims 1 or 2, wherein the Cas protein is Cas12.

4. The method of any of claims 1 to 3, wherein it is determined whether the Cas protein has cleaved an amplified E6 (SEQ ID NO:1) and / or E7 DNA (SEQ ID NO:2).

5. The method of claim 4, wherein the presence of a cleavage product of E6 and / or E7 DNA is determined.

6. The method of claim 4, wherein the determination of a cleavage of E6 and / or E7 DNA is performed by target-activated, non-specific single-stranded deoxyribonuclease cleavage by Cas12.

7. The method of claim 6, wherein the target-activated, non-specific single-stranded deoxyribonuclease cleavage by Cas12 is determined using a kit.

8. The method of any preceding claim, wherein the E6 and / or E7 specific primer has the following sequence(s):
E6: HPV16-E6-F GCACCAAAAGAGAACTGCAATG (SEQ ID NO: 3);
HPV16- E6- R GTTTGCAGCTCTGTGCATAACTG (SEQ ID NO: 4);
E7: HPV16- E7- F CAGCTCAGAGGAGGAGGATG (SEQ ID NO: 5);
HPV16- E7- R GTAATGGGCTCTGTCCGGT(SEQ ID NO: 6).

9. The method of any preceding claim, wherein the guide E6 and / or E7 specific guide RNA has the following sequence(s):
E6: UAAUUUCUACUAAGUGUAGAUUGGGUCGCUCCUGUGGGUCC(SEQ ID NO: 7),
E7: UAAUUUCUACUAAGUGUAGAUAUCCUCCUCCUCUGAGCUGU (SEQ ID NO: 8).

10. A kit comprising a Cas protein, a nucleic acid, a specific guide RNA, buffers and detection means, for determining the cleavage of E6 and / or E7 DNA in a body fluid.

11. The kit of claim 10, wherein the kit is for determining the cleavage in a method according to any of claims 1 to 9.

12. Use of the kit of claim 10 for diagnosing and/or monitoring cancer in a subject.
